# EUROPÄISCHE PATENTSCHRIFT

(11) **EP 2 138 204 B1**
(45) Veröffentlichungstag und Bekanntmachung des Hinweises auf die Patenterteilung: **04.01.2012**
(21) Anmeldenummer: 09007462.6
(22) Anmeldetag: 05.06.2009
(51) Int. Cl.: A61N 2/02, G01C 17/08

(54) **Vorrichtung zur Behandlung von Arthrose zumindest unter Einsatz von pulsierenden Magnetfeldern**
Device for treating arthrosis, at least when using pulsing magnetic fields
Dispositif de traitement de l'arthrose, au moins par l'utilisation de champs magnétiques pulsés

(30) Priorität: 25.06.2008 DE 102008029860
(43) Veröffentlichungstag der Anmeldung: 30.12.2009
(73) Patentinhaber: Medisana AG, 40724 Hilden (DE)
(72) Erfinder: Lindner, Rolf, 40597 Düsseldorf (DE)
(74) Vertreter: Ostriga, Sonnet, Wirths & Roche

(56) Entgegenhaltungen:
- EP-A- 0 373 141
- EP-A- 0 503 307
- GB-A- 363 287
- US-A- 1 614 228
- US-A- 5 079 846

## Beschreibung

Die Erfindung betrifft eine Vorrichtung zur Behandlung von Arthrose zumindest unter Einsatz von pulsierenden Magnetfeldern, wenigstens bestehend aus einer Einheit zur Erzeugung von pulsierenden Magnetfeldern und einer Steuereinheit, wobei das Steuergerät eine optische Betriebsanzeige aufweist.

Derartige Geräte, insbesondere zur Arthrosebehandlung sind allgemein bekannt und erzeugen häufig neben pulsierenden Magnetfeldern auch Wärme.

Aufgrund der Erfahrungen zahlreicher Studien im In- und Ausland konnten in den letzten Jahren hoch spezialisierte Geräte entwickelt werden, die nachweislich heilend wirkende Magnetfelder aufbauen. Durch den Einsatz der pulsierenden Magnettherapie konnte beispielsweise eine erhöhte Durchlässigkeit von Teilchen an den Zellmembranen nachgewiesen werden, wodurch ein vergrößerter Austausch von Nähr- und Abfallstoffen entstand. Außerdem erhöhte sich der Sauerstoffgehalt im Gewebe, was eine bessere Sauerstoffausnutzung der Zelle zur Folge hat und die Immunabwehr stimuliert und dadurch auch entzündungshemmend wirkt. Chronische Entzündungsprozesse können durch Magnetfelder maßgeblich positiv beeinflusst werden. Letztlich fand man auch eine Beschleunigung der Genesung bei schlecht heilenden Knochenbrüchen, wenn man diese speziellen Magnetfeldern aussetzte. Die Wirkfaktoren eines pulsierenden Magnetfeldes hängen von der Frequenz der magnetischen Wellen und deren Stärke ab.

Während der Nutzer eines derartigen Gerätes sofort die Einwirkung der Wärme auf dem Körperteil wahrnimmt, kann er bei den bekannten Geräten lediglich durch eine optische Betriebsanzeige nach Art einer LED erkennen, dass die Vorrichtung zur Erzeugung eines pulsierenden Magnetfeldes in Betrieb ist.

Die Aufgabe der Erfindung besteht nun darin, eine neue optische Betriebsanzeige zu schaffen, welche dem Nutzer wesentlich deutlicher den Betriebszustand der Einheit zur Erzeugung von pulsierenden Magnetfeldern anzeigt.

Die Lösung dieser Aufgabe ergibt sich aus den Merkmalen des Anspruches 1, insbesondere den Merkmalen des Kennzeichenteils, wonach die optische Betriebsanzeige aus einem frei beweglich, in einem durchsichtigen Hohlkörper angeordneten Permanentmagnet gebildet wird, der sich während des Betriebs der Vorrichtung innerhalb des pulsierenden Magnetfeldes bewegt.

Zusätzlich werden durch die Bewegung des Permanentmagneten im pulsierenden Magnetfeld auch akustische Signale erzeugt. Dies verstärkt zusätzlich die vorgenannten psychologischen Wirkungen einerseits. Andererseits können auch sehbehinderte oder blinde Nutzer der Vorrichtung wahrnehmen, dass pulsierende Magnetfelder auf einen Körperteil einwirken.

Der erfindungsgemäße Vorteil dieser optischen Betriebsanzeige besteht darin, dass dem Nutzer wesentlich deutlicher gemacht wird, dass zum Zwecke der Behandlung, beispielsweise der Arthrose, während des Behandlungszeitraumes pulsierende Magnetfelder auf die entsprechenden Körperteile einwirken, wodurch die medizinischen Wirkungen der pulsierenden Magnetfeldtherapie durch psychologische Wirkungen verstärkt werden.

Letztlich ist es auch möglich, dass die Intensität der Bewegung des Permanentmagnetes von der Stärke des pulsierenden Magnetfeldes gesteuert wird.

Weitere Vorteile der Erfindung ergeben sich aus den weiteren Unteransprüchen sowie der nachfolgenden Beschreibung eines Ausführungsbeispieles. Es zeigen:
Fig. 1 Darstellung einer Vorrichtung zur Behandlung von Arthrose und
Fig. 2 eine vergrößerte, mit II gekennzeichnete Teildarstellung der Betriebsanzeige der Vorrichtung gemäß Fig. 1.

In den Zeichnungen ist eine Vorrichtung zur Behandlung von insbesondere Arthrose insgesamt mit der Bezugsziffer 10 bezeichnet.

Eine derartige Vorrichtung wird aus einer Manschette 11 mit einem Stromleiter 12 und einer Einheit 13 zur Erzeugung von pulsierenden Magnetfeldern gebildet, welche mit einem Stromanschluss 14 versehen ist.

Auf nicht dargestellte Weise ist meist eine derartige Vorrichtung 10 zusätzlich mit einer Wärmequelle versehen.

Die Einheit 13 zur Erzeugung von pulsierenden Magnetfeldern weist einen Ein-/Ausschalter S und eine Betriebsanzeige 15 auf, welche aus einer Kavität 16 gebildet wird, die von einem durchsichtigen Hohlkörper 17 abgedeckt wird. Innerhalb der Kavität 16 ist ein Permanentmagnet 18 frei beweglich angeordnet.

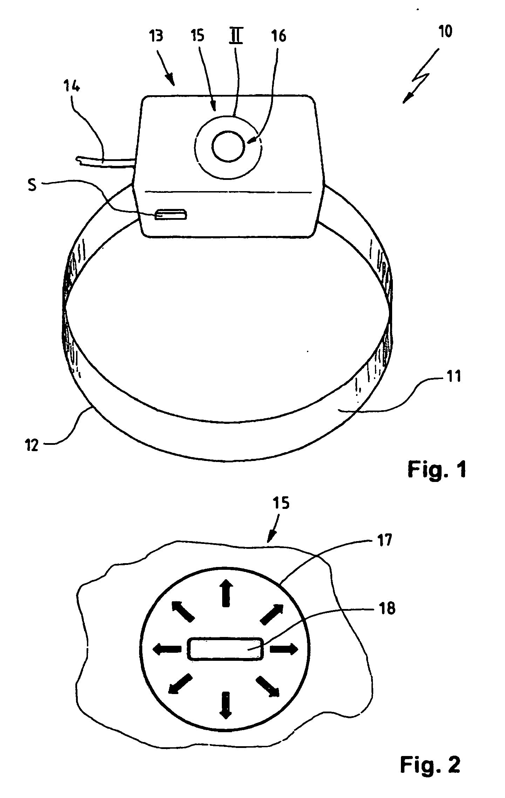

In der Fig. 2 ist erkennbar, dass der Permanentmagnet 18 während des Betriebes der Vorrichtung 10 unter Einwirkung des pulsierenden Magnetfeldes sich frei innerhalb der Kavität 16 sowie dem durchsichtigen Hohlkörper 17 bewegt.

Dadurch erkennt der Benutzer, dass das pulsierende Magnetfeld auf ein bestimmtes Körperteil einwirkt.

## Patentansprüche

1. Vorrichtung zur Behandlung von insbesondere Arthrose zumindest unter Einsatz von pulsierenden Magnetfeldern, wenigstens bestehend aus einer Einheit zur Erzeugung von pulsierenden Magnetfeldern und einer Steuereinheit, wobei das Steuergerät eine optische Betriebsanzeige aufweist, **dadurch gekennzeichnet, dass** die optische Betriebsanzeige (15) aus einem frei beweglich in einem durchsichtigen Hohlkörper (17) angeordneten Permanentmagnet (18) gebildet wird, der während des Betriebs der Vorrichtung (10) innerhalb des pulsierenden Magnetfeldes bewegbar ist und dass die Bewegung des Permanentmagneten (18) im pulsierenden Magnetfeld auch akustische Signale erzeugt.

2. Vorrichtung nach Anspruch 1, **dadurch gekennzeichnet, dass** die Intensität der Bewegungen des Permanentmagnetes (18) von der Stärke des pulsierenden Magnetfeldes gesteuert wird.

3. Vorrichtung nach einem der vorangehenden Ansprüche, **dadurch gekennzeichnet, dass** die Einheit (13) zur Erzeugung von pulsierenden Magnetfeldern aus einer an einem Körperteil befestigbaren Manschette (11) gebildet wird, in der ein stromdurchflossener Leiterdraht (12) angeordnet ist.

4. Vorrichtung nach einem der Ansprüche 1 bis 2, **dadurch gekennzeichnet, dass** die Einheit zur Erzeugung von pulsierenden Magnetfeldern in einer Matte angeordnet ist.

## Claims

1. Device for treating, in particular, osteoarthritis, at least using pulsating magnetic fields, at least consisting of a unit for generating pulsating magnetic fields and a control unit, the controller having an optical operation indicator, **characterized in that** the optical operation indicator (15) is formed by a permanent magnet (18) which is arranged freely movably in a transparent hollow body (17), and which can be moved within the pulsating magnetic field during operation of the device (10), and **in that** the movement of the permanent magnet (18) in the pulsating magnetic field also generates acoustic signals.

2. Device according to Claim 1, **characterized in that** the intensity of the movements of the permanent magnet (18) is controlled by the strength of the pulsating magnetic field.

3. Device according to any one of the preceding claims, **characterized in that** the unit (13) for generating pulsating magnetic fields is formed by a collar (11) which can be fixed to a body part, and in which a conductive wire (12), through which a current flows, is arranged.

4. Device according to one of Claims 1 to 2, **characterized in that** the unit for generating pulsating magnetic fields is arranged in a mat.

## Revendications

1. Dispositif pour le traitement d'arthroses particulières, au moins par utilisation de champs magnétiques pulsés, comprenant au moins une unité pour la production de champs magnétiques pulsés et une unité de commande, l'appareil de commande présentant un indicateur optique de fonctionnement, **caractérisé en ce que** l'indicateur optique de fonctionnement (15) est formé d'un aimant permanent (18) déplaçable librement, disposé dans un corps creux (17) transparent qui peut se déplacer au cours du fonctionnement du dispositif (10) à l'intérieur du champ magnétique pulsé et **en ce que**, dans le champ magnétique pulsé, le déplacement de l'aimant permanent (18) crée également des signaux acoustiques.

2. Dispositif selon la revendication 1, **caractérisé en ce que** l'intensité des déplacements de l'aimant permanent (18) est commandée par l'intensité du champ magnétique pulsé.

3. Dispositif selon l'une des précédentes revendications, **caractérisé en ce que** l'unité (13) pour la production de champs magnétiques pulsés est formée d'une manchette (11) qui peut être fixée au niveau d'une partie du corps et dans laquelle est disposé un fil conducteur (12) parcouru par le courant.

4. Dispositif selon l'une des revendications 1 à 2, **caractérisé en ce que** l'unité pour la production de champs magnétiques pulsés est disposée dans un feutre.
